# EUROPÄISCHE PATENTSCHRIFT

(11) **EP 1 119 531 B1**
(45) Veröffentlichungstag und Bekanntmachung des Hinweises auf die Patenterteilung: **25.08.2004**
(21) Anmeldenummer: 99948927.1
(22) Anmeldetag: 04.10.1999
(51) Int. Cl.: C07C 2/08, C07C 7/12

(54) **VERFAHEN ZUR KATALYTISCHEN OLIGOMERISIERUNG VON MONOOLEFINEN**
CATALYTIC MONOOLEFIN OLIGOMERIZATION PROCESS
PROCEDE D'OLIGOMERISATION CATALYTIQUE DE MONO-OLEFINES

(30) Priorität: 05.10.1998 DE 19845857
(43) Veröffentlichungstag der Anmeldung: 01.08.2001
(73) Patentinhaber: BASF AKTIENGESELLSCHAFT, 67056 Ludwigshafen (DE)
(72) Erfinder: MÜLLER, Ulrich, D-67435 Neustadt (DE); WALTER, Marc, D-67227 Frankenthal (DE); BROX, Wolfgang, D-69118 Heidelberg (DE)
(74) Vertreter: Kinzebach, Werner, Dr.
(86) Internationale Anmeldenummer: PCT/EP1999/007331
(87) Internationale Veröffentlichungsnummer: WO 2000/020359

(56) Entgegenhaltungen:
- DE-A- 2 135 437
- DE-A- 3 612 443
- DE-B- 1 197 872
- US-A- 4 188 501

## Beschreibung

Die vorliegende Erfindung betrifft ein Verfahren zur katalytischen Oligomerisierung von in einem Kohlenwasserstoffgemisch enthaltenen kurzkettigen Monoolefinen, wobei man das Gemisch mit einem Adsorptionsmittel in Kontakt bringt und anschließend katalytisch oligomerisiert.

Kohlenwasserstoffgemische, die kurzkettigen Olefine, z. B. mit 2 bis 8 Kohlenstoffatomen enthalten, sind im großtechnischen Maßstab erhältlich. So fällt z. B. bei der Aufarbeitung von Erdöl durch Steamcracken oder Fluidized Catalyst Cracking (FCC) ein als C₄-Schnitt bezeichnetes Kohlenwasserstoffgemisch mit einem hohen Gesamtolefingehalt an, wobei es sich im Wesentlichen um Olefine mit 4 Kohlenstoffatomen handelt. Solche C₄-Schnitte, d. h. Gemische aus isomeren Butenen und Butanen, eignen sich, gegebenenfalls nach einer vorherigen Abtrennung des Isobutens und Hydrierung des enthaltenen Butadiens, sehr gut zur Herstellung von Oligomeren, insbesondere von Octenen und Dodecenen.

Eine große Bedeutung haben die aus Olefingemischen mit überwiegend linearen Ausgangsolefinen erhältlichen linearen Oligomerengemische erlangt. Sie eignen sich z. B. als Dieselkraftstoffkomponente sowie als Zwischenprodukte zur Herstellung funktionalisierter, überwiegend linearer Kohlenwasserstoffe. So erhält man durch Hydroformylierung und anschließende Hydrierung der Olefinoligomere die entsprechenden Alkohole, die unter anderem als Ausgangsstoffe für Detergenzien und als Weichmacher verwendet werden. Für einen Einsatz als Weichmacheralkohole spielt der Verzweigungsgrad der Olefine eine entscheidende Rolle. Der Verzweigungsgrad wird dabei durch den ISO-Index beschrieben, der die mittlere Zahl der Methylverzweigungen der jeweiligen Olefinfraktion angibt. So tragen z. B. bei einer C₈-Fraktion die n-Octene mit 0, Methylheptene mit 1 und Dimethylhexene mit 2 zum ISO-Index der Fraktion bei. Je niedriger der ISO-Index ist, um so größer ist die Linearität der Moleküle in der jeweiligen Fraktion. Je höher die Linearität ist, um so höher sind im Allgemeinen die Ausbeuten bei der Hydroformylierung und um so besser sind die Eigenschaften der daraus hergestellten Weichmacher.

Zur großtechnischen Herstellung von Olefinoligomeren allgemein und speziell von wenig verzweigten Olefinen aus olefinhaltigen Kohlenwasserstoffgemischen sind sowohl homogen als auch heterogen katalysierte Verfahren bekannt. Ein Nachteil der homogen katalysierten Verfahren besteht in der technisch aufwendigen Abtrennung des Katalysators vom Reaktionsgemisch. Bei dieser Abtrennung fallen Rückstände an, die entweder aufwendig aufgearbeitet oder entsorgt werden müssen, da sich die eingesetzten homogenen Katalysatoren im Allgemeinen nicht regenerieren lassen. Somit sind homogen katalysierte Verfahren gegenüber heterogen katalysierten wirtschaftlich benachteiligt.

Die bekannten heterogenen Katalysatorsysteme basieren im Wesentlichen auf Nickel- und Silicium-haltigen Katalysatoren, die oftmals zusätzlich noch Aluminium enthalten. verfahren zur Herstellung dieser Katalysatoren und deren Einsatz zur Olefinoligomerisierung sind bekannt.

Die DE-A-43 39 713 beschreibt ein Verfahren zur katalytischen Oligomerisierung von unverzweigten C₂- bis C₆-Olefinen an einem Festbettkatalysator, der als wesentliche aktive Bestandteile 10 bis 70 Gew.-% Nickeloxid, 5 bis 30 Gew.-% Titandioxid und/oder Zirkonoxid, 0 bis 20 Gew.-% Aluminiumoxid und als Differenz zu 100 Gew.-% Siliciumoxid enthält. Dabei erhält man in guten Ausbeuten überwiegend lineare Oligomere, d. h. Oligomere mit einem niedrigen ISO-Index.

Die zur Oligomerisierung eingesetzten, großtechnisch zugängigen Olefingemische enthalten oftmals Verbindungen, die als Katalysatorgifte wirken und den Oligomerisierungskatalysator deaktivieren. Zu diesen Katalysatorgiften zählen z. B. Alkine und mehrfach ungesättigte Kohlenwasserstoffe, wie Butadien. Dazu zählen weiterhin sauerstoffhaltige Verbindungen, wie Alkohole, Aldehyde, Ketone und Ether sowie stickstoffhaltige, schwefelhaltige und halogenhaltige Verbindungen. Die Anwesenheit solcher Katalysatorgifte bei der Oligomerisierung führt im Laufe der Zeit zu einer Abnahme der Katalysatoraktivität, die häufig irreversibel ist.

Aus dem Stand der Technik sind Verfahren bekannt, um einzelne, die Oligomerisierung beeinträchtigende Komponenten aus einem Kohlenwasserstoffgemisch zu entfernen. So beschreibt z. B. die DE-A-20 57 269 ein Oligomerisierungsverfahren, bei dem aus dem eingesetzten Kohlenwasserstoffgemisch zunächst mehrfach ungesättigte Olefine durch katalytische Hydrierung zu den entsprechenden Monoolefinen entfernt werden. weiterhin ist z. B. bekannt, Schwefelverbindungen durch eine Alkaliwäsche und Stickstoffverbindungen durch eine Wasserwäsche aus olefinhaltigen Kohlenwasserstoffgemischen, die zur Oligomerisierung eingesetzt werden, zu entfernen. Die zuvor genannten Verfahren eignen sich dabei jedoch nur zu einer groben Entfernung von als Katalysatorgifte wirksamen Verbindungen. Da viele Katalysatorgifte bereits in Spurenmengen die Lebenszeit eines Oligomerisierungskatalysators verringern können, reichen die üblichen Verfahren zu ihrer Entfernung im Allgemeinen nicht aus, um geeignete Olefingemische für die Oligomerisierung zu gewinnen, die gute Standzeiten der eingesetzten Katalysatoren ermöglichen.

Die DE-B 11 97 872 beschreibt ein Verfahren zur Reinigung von C₆-C₁₈-Spaltwachs- oder -Spaltraffinatolefinen mit einer Aufschlämmung von erhitztem Siliciumdioxid.

Die DE-A 21 35 437 beschreibt ein Verfahren zur Reinigung von flüssigem Propen durch Behandlung mit mineralischen Absorptionsmitteln, wie Aluminiumoxid, Siliziumdioxid und/oder Eisenoxid.

Die DE-A 36 12 443 beschreibt ein Verfahren zur Herstellung von Tetraisobutylen durch katalytische Dimerisierung von Diisobutylen. Das Edukt wird vor der Oligomerisierung mit einem regenerierbaren anorganischen Absorptionsmittel, das ausgewählt ist unter aktivierten Kieselgelen, Aluminiumoxid-Gelen und Molekularsieb-Zeolithen, vorbehandelt.

Die DE-A-39 14 817 beschreibt ein Verfahren zur Olefinoligomerisierung an einem heterogenen nickelhaltigen Festbettkatalysator, wobei man das Einsatz-Kohlenwasserstoff-Gemisch vor der Oligomerisierung über ein Molekularsieb mit einem Porendurchmesser von größer als 4 Ångström bis 15 Ångström leitet und die enthaltenen, mehrfach ungesättigten Kohlenwasserstoffe durch eine selektive Hydrierung entfernt. Bei den eingesetzten Molekularsieben handelt es sich dabei ausschließlich um natürliche und synthetische Aluminiumsilikate, d. h. um Zeolithe. Dies entspricht der Definition des Begriffes "Molekularsieb" als ein Zeolith mit starkem Adsorptionsvermögen, gemäß Römpp, Chemie-Lexikon, 9. Auflage (Paperback), Band 4, S. 2829. Der Einsatz von anderen Adsorptionsmaterialien als von Zeolithen wird in diesem Dokument nicht beschrieben. Der Einsatz von Aluminiumsilikaten zur Adsorption von als Katalysatorgiften wirksamen Verunreinigungen aus olefingemischen für die katalytische Oligomerisierung ist mit mehreren Nachteilen verbunden. So nimmt die zur Adsorption zur Verfügung stehende Oberfläche von Molekularsieben in Gegenwart von Wasserdampf irreversibel ab. Die Desorption sauerstoffhaltiger Verunreinigungen zur Regenerierung des gesättigten Adsorptionsmittels erfolgt jedoch im Allgemeinen durch Behandlung mit wasserdampf bei erhöhten Temperaturen. Auch bei der Regenerierung des gesättigten Adsorptionsmittels durch Behandlung mit Sauerstoff bei erhöhten Temperaturen wird Wasserdampf gebildet. Zudem enthalten die zur Oligomerisierung eingesetzten technischen Olefingemische gewisse Mengen an Wasser, welches bei einem Einsatz zeolithischer Adsorptionsmittel in einem zusätzlichen Verfahrensschritt aufwendig entfernt werden muss. Zeolithe neigen unter thermischer Belastung, wie sie z. B. üblicherweise bei der Regenerierung von Adsorptionsmitteln auftritt, zur Ausbildung Lewis-saurer Gruppen. Diese Gruppen katalysieren die Oligomerisierung von Olefinen, so dass es bereits bei der Reinigung der Olefinedukte zu einer unkontrollierten Oligomerenbildung kommt. Dies führt zum einen zu einer Abnahme der Selektivität des Verfahrens bezüglich einzelner erwünschter Oligomere, die Produkte zeigen eine im Allgemeinen unerwünscht breite Molekulargewichtsverteilung. Dies führt zum anderen auch zu einem unerwünscht hohen Verzweigungsgrad (ISO-Index) der erhaltenen Oligomere, die sich somit für eine Weiterverarbeitung zu Weichmachern nicht eignen. Eine durch das Adsorptionsmittel katalysierte Oligomerenbildung führt schließlich zur Ablagerung von höher siedenden Kohlenwasserstoff-Verbindungen auf der Adsorberoberfläche, was eine beschleunigte Abnahme der Adsorptionsleistung und eine verminderte Steilheit der Durchbruchskurve des Adsorbers nach sich zieht. Das zeolithische Adsorptionsmittel muss in immer kürzeren Abständen regeneriert werden, wobei aus den abgelagerten, höher siedenden Kohlenwasserstoffen dabei in der Regel Kohlendioxid und Wasser gebildet werden. Letzteres führt, wie erwähnt, zu einer Abnahme der zur Adsorption zur Verfügung stehenden Oberfläche und fördert die Bildung von Lewissauren Gruppen. Zeolithische Molekularsiebe weisen somit mit jedem Regenerierungsschritt eine abnehmende Zykluslänge auf. Dies führt zu steigenden Betriebskosten und somit einer wirtschaftlichen Benachteiligung der auf ihnen basierenden Verfahren.

Der vorliegenden Erfindung liegt die Aufgabe zugrunde, ein Verfahren zur katalytischen Oligomerisierung von Monoolefinen zur Verfügung zu stellen, bei dem das Eduktolefingemisch zur Entfernung von enthaltenen Katalysatorgiften mit einem Adsorptionsmittel in Kontakt gebracht wird, das sich einfach und im Wesentlichen ohne Abnahme der Adsorptionskapazität regenerieren lässt.

Überraschenderweise wurde nun gefunden, dass diese Aufgabe gelöst wird, wenn man ein Adsorptionsmittel einsetzt, das ausgewählt ist unter Aluminiumoxiden und aluminiumoxidhaltigen Feststoffen.

Gegenstand der vorliegenden Erfindung ist daher ein Verfahren zur katalytischen Oligomerisierung von in einem Kohlenwasserstoffgemisch enthaltenen kurzkettigen Monoolefinen, wobei man das Gemisch mit einem Adsorptionsmittel in Kontakt bringt und katalytisch oligomerisiert und wobei ein Kohlenwasserstoffgemisch eingesetzt wird, das mindestens ein Monoolefin mit 4 bis 8 Kohlenstoffatomen aufweist und das ausschließlich oder einen hohen Anteil unverzweigte(r) Monoolefine enthält und wobei als Adsorptionsmittel ein Aluminiumoxid oder ein aluminiumoxidhaltiger Feststoff eingesetzt wird.

Geeignete Adsorptionsmittel für das erfindungsgemäße Verfahren sind allgemein übliche und dem Fachmann bekannte sog. aktive Aluminiumoxide. Ihre Herstellung erfolgt z. B. ausgehend von Aluminiumhydroxid, welches durch übliche Fällungsverfahren aus Aluminiumsalzlösungen erhältlich ist. Dazu zählt z. B. das kommerziell erhältliche Bayer-Aluminiumhydroxid. Diese Aluminiumhydroxide können dann direkt durch Erhitzen auf Temperaturen von etwa 700 bis 2 100 °C in geeigneten Öfen, wie z. B. Dreh- oder Fließbett-Kalzinieröfen, zu aktiven Aluminiumoxiden umgesetzt werden. Zur Herstellung von kugelförmigem aktiviertem Aluminiumoxid kann Aluminiumhydroxid einer Stoßerhitzung im Vakuum oder in Gegenwart eines hoch temperaturbeständigen Gases unterzogen werden. Dabei wird das Aluminiumhydroxid für kurze Zeit, im Allgemeinen wenige Sekunden, auf Temperaturen von etwa 1 100 bis 1 600 °C erhitzt. Für das erfindungsgemäße Verfahren geeignete aktive Aluminiumoxide sind auch ausgehend von Aluminiumhydroxid-Gelen erhältlich. Zur Herstellung solcher Gele kann z. B. gefälltes Aluminiumhydroxid nach üblichen Aufarbeitungsschritten, wie Filtern, Waschen und Trocknen, aktiviert und anschließend gegebenenfalls Vermahlen oder agglomeriert werden. Gewünschtenfalls kann man das resultierende Aluminiumoxid anschließend noch einem Formgebungsverfahren, wie Extrusion, Granulation, Tablettierung etc. unterwerfen. Als Adsorptionsmittel eignen sich vorzugsweise die Selexsorb®-Typen der Fa. Alcoa.

Geeignete Adsorptionsmittel für das erfindungsgemäße Verfahren sind weiterhin aluminiumoxidhaltige Feststoffe. Dazu zählen z. B. die sogenannten Tonerden, die als Hauptbestandteil ebenfalls Aluminiumoxide aufweisen. Dazu zählen weiterhin z. B. Gemische aus wenigstens einem Aluminiumoxid und wenigstens einem weiteren der zuvor genannten Adsorptionsmittel.

Gewünschtenfalls können die erfindungsgemäß eingesetzten Aluminiumoxide oder aluminiumoxidhaltigen Feststoffe zusätzlich wenigstens eine Alkalimetall- und/oder Erdalkalimetallverbindung aufweisen. Zu diesem Zweck kann das Aluminiumoxid oder der aluminiumoxidhaltige Feststoff z. B. einem Tränkschritt mit einer entsprechenden Lösung und gegebenenfalls weiteren üblichen Verfahrensschritten, wie Trocknen und/oder Kalzinieren, unterzogen werden. Vorzugsweise erfolgt diese Modifizierung des Adsorptionsmittels nach der Formgebung. Bevorzugt weist das Aluminiumoxid oder der aluminiumoxidhaltige Feststoff einen Alkalimetall- und/oder Erdalkalimetallgehalt von höchstens 1 Gew.-%, bezogen auf das Gesamtgewicht des Adsorptionsmittels, auf. Dieser liegt dann z. B. in einem Bereich von etwa 0,0001 bis 1,0 Gew.-%. vorzugsweise enthalten die erfindungsgemäß eingesetzten Aluminiumoxide oder aluminiumoxidhaltigen Feststoffe die Alkalimetalle und/oder Erdalkalimetalle in Form ihrer Oxide.

Erfindungsgemäß wird als Adsorptionsmittel in dem erfindungsgemäßen Verfahren wenigstens ein Aluminiumoxid oder ein aluminiumoxidhaltiger Feststoff eingesetzt.

Besonders bevorzugt sind Aluminiumoxide und aluminiumoxidhaltige Feststoffe, die wenigstens ein Alkalimetall- und/oder Erdalkalimetall aufweisen. Vorzugsweise beträgt der Alkalimetall- und/oder Erdalkalimetallgehalt höchstens 1 Gew.-%, z. B. 0,0001 bis 1 Gew.-%, bezogen auf das Gesamtgewicht des Adsorptionsmittels.

Bevorzugt wird ein Adsorptionsmittel mit einer spezifischen Oberfläche, bestimmt nach BET, im Bereich von etwa 10 bis 2 000 m²/g, bevorzugt 10 bis 1 500 m²/g, insbesondere 20 bis 600 m²/g, eingesetzt.

Bevorzugt weisen die erfindungsgemäß eingesetzten Adsorptionsmittel selbst im Wesentlichen keine katalytische Aktivität zur Oligomerisierung von Olefinen auf. So beobachtet man bei einem erstmals für das erfindungsgemäße Verfahren eingesetzten erfindungsgemäßen Adsorptionsmittel (sogenannter 1. Zyklus) eine Oligomerenbildung von höchstens 0,2 Gew.-%, bevorzugt höchstens 0,1 Gew.-%, bezogen auf die Gesamtmonoolefinmenge in dem zur Oligomerisierung eingesetzten Kohlenwasserstoffgemisch. Vorteilhafterweise nimmt diese Tendenz zur Oligomerenbildung auch bei regenerierten Adsorptionsmitteln (2. bis n. Zyklus) im Wesentlichen nicht zu.

Die erfindungsgemäß eingesetzten Adsorptionsmittel eignen sich ganz allgemein zur Behandlung üblicher monoolefinhaltiger Kohlenwasserstoffgemische, mit dem Ziel den darin enthaltenen Anteil an Komponenten, die die katalytische Oligomerisierung durch Deaktivierung des eingesetzten Oligomerisierungskatalysators beeinträchtigen, sogenannten Katalysatorgiften, zu verringern. Zu den üblicherweise in solchen Kohlenwasserstoffgemischen enthaltenen Katalysatorgiften zählen sauerstoffhaltige Verbindungen, z. B. Wasser, Alkohole, wie Methanol, Ethanol, Isopropanol, n-Propanol, n-Butanol, sec.-Butanol, Isobutanol, tert.-Butanol, Ether, wie z. B. Diethylether, Methyl-tert.-butylether, Aldehyde, wie z. B. Formaldehyd, Acetaldehyd, Propionaldehyd, Butyraldehyd, Ketone, wie z. B. Aceton, Methylethylketon, etc. Weitere üblicherweise enthaltene Katalysatorgifte sind stickstoffhaltige Verbindungen, wie z. B. Amine, und schwefelhaltige Verbindungen, wie z. B. Schwefelwasserstoff, Thioalkohole, Thioether, wie z. B. Dimethylsulfid und Dimethyldisulfid, Kohlenoxidsulfid etc. Gegebenenfalls können großtechnisch zur Olefinoligomerisierung eingesetzte Kohlenwasserstoffgemische auch noch weitere produktionsbedingte Katalysatorgifte enthalten. Dazu zählen z. B. Halogenverbindungen, Spuren von üblichen Extraktionsmitteln, wie z. B. Acetonitril oder N-Methylpyrrolidon etc., sowie gegebenenfalls organische Phosphor- und Arsenverbindungen.

Der Gehalt an Katalysatorgiften in den im erfindungsgemäßen Verfahren eingesetzten Kohlenwasserstoffgemischen ist prinzipiell kein limitierender Faktor für den Einsatz der zuvor beschriebenen Adsorptionsmittel. Mit zunehmendem Katalysatorgiftgehalt nimmt jedoch das erforderliche Einsatzvolumen des Adsorptionsmittels pro Zeiteinheit und Kohlenwasserstoffdurchsatz zu. Daher kann es unter Umständen wirtschaftlicher sein, die zur Olefinoligomerisierung eingesetzten Kohlenwasserstoffgemische vor dem In-Kontakt-bringen mit den zuvor beschriebenen Adsorptionsmitteln einer Grobreinigung nach aus dem Stand der Technik bekannten Verfahren zu unterziehen. So ist es im Allgemeinen günstig, den Katalysatorgiftgehalt in den Kohlenwasserstoffgemischen erforderlichenfalls vor dem In-Kontakt-bringen mit den erfindungsgemäß eingesetzten Adsorptionsmitteln durch übliche Verfahren auf höchstens etwa 5 000 Gew.-ppm, bevorzugt höchstens 2 000 Gew.-ppm, insbesondere höchstens 1 000 Gew.-ppm, zu verringern. Geeignete Verfahren zur Grobreinigung von Kohlenwasserstoffgemischen sind z. B. in der DE-A-39 14 817 und der darin zitierten Literatur beschrieben, auf die hier in vollem Umfang Bezug genommen wird.

Vorzugsweise wird durch das Behandeln der Kohlenwasserstoffgemische mit den zuvor beschriebenen Adsorptionsmitteln ein monoolefinhaltiges Einsatz-Kohlenwasserstoff-Gemisch für die Oligomerisierung erhalten, das einen Gehalt an sauerstoff-, stickstoffund/oder schwefelhaltigen Verunreinigungen von höchstens 10 ppm, bevorzugt höchstens 1 ppm, aufweist.

Die Adsorptionsmittel können vor ihrem Einsatz in dem erfindungsgemäßen Verfahren einem üblichen Formgebungsverfahren, wie z. B. Pelletieren, Tablettieren oder Extrudieren unterzogen werden. Vorzugsweise werden die Adsorptionsmittel in Form von Extrudaten oder von Kugeln eingesetzt. Kugelförmige Adsorptionsmittel weisen vorzugsweise einen Durchmesser im Bereich von etwa 0,1 bis 5 mm auf.

Geeignete, gegebenenfalls druckfeste Reaktionsapparaturen für das In-Kontakt-bringen des Kohlenwasserstoffgemisches mit den zuvor beschriebenen Adsorptionsmitteln sind dem Fachmann bekannt. Dazu zählen die allgemein üblichen Reaktoren für Gas/Fest-Reaktionen und Flüssig/Fest-Reaktionen. Bevorzugt wird ein Festbettreaktor oder ein wanderbettreaktor eingesetzt. Gewünschtenfalls kann das Adsorptionsmittel in Form einer einzigen oder in Form mehrerer Kontaktzonen eingesetzt werden. Dabei kann jede dieser Zonen eines oder mehrere der zuvor beschriebenen Adsorptionsmittel aufweisen. Adsorption und Oligomerisierung können in verschiedenen Reaktoren oder in aufeinanderfolgenden Zonen in einem Reaktor durchgeführt werden. Dabei kann es dann unter Umständen erforderlich sein, dass die Zonen für die Adsorption und die Zonen für die Oligomerisierung bei unterschiedlichen Reaktionsbedingungen, vorzugsweise bei unterschiedlichen Temperaturen, betrieben werden.

Das In-Kontakt-bringen des Kohlenwasserstoffgemisches mit dem Adsorptionsmittel erfolgt im Allgemeinen durch Überleiten. Dabei kann das Kohlenwasserstoffgemisch gewünschtenfalls in gasförmiger, flüssiger oder überkritischer Phase mit dem Adsorptionsmittel in Kontakt gebracht werden. Die lineare Geschwindigkeit liegt dabei vorzugsweise in einem Bereich von etwa 1 bis 200 cm/min.

Vorzugsweise wird das Kohlenwasserstoffgemisch bei einer Temperatur im Bereich von etwa 0 bis 150 °C, bevorzugt 5 bis 100 °C, insbesondere 10 bis 50 °C, mit dem Adsorptionsmittel in Kontakt gebracht.

Vorzugsweise wird das Kohlenwasserstoffgemisch bei einem Druck im Bereich von etwa 1 bis 200 bar, bevorzugt etwa 1 bis 100 bar, insbesondere etwa 1 bis 50 bar, und speziell etwa 2 bis 20 bar, mit dem Adsorptionsmittel in Kontakt gebracht.

Die Regenerierung der erfindungsgemäß eingesetzten Adsorptionsmittel erfolgt vorzugsweise durch Behandlung mit Sauerstoff, sauerstoffhaltigen Gasgemischen, Sauerstoff liefernden Verbindungen, Stickoxiden oder Gemischen davon. Die Temperatur bei der Regenerierung liegt vorzugsweise in einem Bereich von etwa 100 bis 800 °C, insbesondere 120 bis 750 °C.

Vorteilhafterweise können die erfindungsgemäß eingesetzten Adsorptionsmittel häufig regeneriert werden, ohne dass die Adsorptionskapazität merklich nachlässt. Sie eignen sich für eine einfache und wirtschaftliche oxidative thermische Regenerierung. Dabei wird auch nach einer Vielzahl von Regenerierungszyklen im Wesentlichen keine durch das Adsorptionsmittel katalysierte Oligomerisierung beobachtet. Dabei werden im Allgemeinen Zykluszahlen von mindestens 2, bevorzugt mindestens 20, insbesondere mindestens 200 und speziell mindestens 2 000 erreicht.

Eine Regeneration kann des Weiteren auch durch Überleiten von Dampf, Wasserstoff, CO₂, N₂ etc. oder durch eine Wäsche mit einem geeigneten Lösungsmittel erfolgen. Dabei werden im Allgemeinen auch die zuvor genannten Zykluszahlen erreicht.

Die gegebenenfalls in dem zur Oligomerisierung eingesetzten Kohlenwasserstoffgemisch enthaltenen mehrfach ungesättigten Kohlenwasserstoffe und/oder Alkine werden vor der Oligomerisierung durch selektive Hydrierung zu den entsprechenden Monoolefinen entfernt. Geeignete verfahren zur selektiven Hydrierung sind z. B. in der DE-A-20 57 269, EP-A-0 081 041 und DE-A-15 68 542 beschrieben, auf die hier in vollem Umfang Bezug genommen wird. Vorzugsweise wird nach der Hydrierung ein Kohlenwasserstoffgemisch erhalten und zur Oligomerisierung eingesetzt, dessen Gehalt an mehrfach ungesättigten Kohlenwasserstoffen und Alkinen höchstens 10 Gew.-ppm, bevorzugt höchstens 1 Gew.-ppm beträgt.

Die Selektivhydrierung kann gewünschtenfalls sowohl vor dem In-Kontakt-bringen des Kohlenwasserstoffgemisches mit dem Adsorptionsmittel als auch danach erfolgen.

Geeignete Kohlenwasserstoffgemische für das erfindungsgemäße Verfahren weisen mindestens ein Monoolefin oder mehrere Monoolefine mit 4 bis 8 Kohlenstoffatomen, vorzugsweise 4 bis 6 Kohlenstoffatomen, auf. Insbesondere handelt es sich um ein Gemisch mit einem hohen Anteil an Monoolefinen mit 4 Kohlenstoffatomen.

Erfindungsgemäß wird ein Kohlenwasserstoffgemisch eingesetzt, das ausschließlich oder einen hohen Anteil unverzweigte Monoolefine enthält. Vorteilhafterweise zeigen die erfindungsgemäß eingesetzten Adsorptionsmittel im Wesentlichen keine Tendenz zur Isomerisierung unverzweigter Monoolefine. Wird dann ein Oligomerisierungskatalysator eingesetzt, der mit guten Ausbeuten zu wenig verzweigten Oligomeren führt, so werden nach dem erfindungsgemäßen Verfahren Oligomere mit einer hohen Linearität, d. h. mit einem niedrigen ISO-Index, erhalten. Diese eignen sich für spezielle Anwendungen, wie z. B. die Weiterverarbeitung zu Weichmachern.

Vorzugsweise wird zur katalytischen Oligomerisierung nach dem erfindungsgemäßen Verfahren ein großtechnisch anfallendes Kohlenwasserstoffgemisch eingesetzt. Dazu zählen z. B. die bei der Aufarbeitung von Erdöl durch Steamcracken oder Fluidized Catalyst Cracking (FCC) erhaltenen C₄-Schnitte. Nach einer speziellen Ausführungsform wird zur Oligomerisierung das sog. Raffinat II eingesetzt.

Nach einer speziellen Ausführungsform des erfindungsgemäßen Verfahrens wird ein großtechnisch anfallender C₄-Schnitt eingesetzt, der 10 bis 90 Gew.-% Butene und 10 bis 90 Gew.-% Butane enthält, wobei die Buten-Fraktion die folgenden Kohlenwasserstoffe enthält:
1 bis 99 Gew.-% trans-2-Buten,
1 bis 50 Gew.-% 1-Buten,
1 bis 50 Gew.-% cis-2-Buten,
1 bis 5 Gew.-% Isobuten.

Zur katalytischen Oligomerisierung der monoolefinhaltigen Kohlenwasserstoffgemische können alle üblichen, dem Fachmann bekannten Oligomerisierungsverfahren eingesetzt werden. Dazu zählt insbesondere das in der DE-A-43 39 713 beschriebene Verfahren, das die Herstellung hochlinearer Oligomere erlaubt. Auf dieses Verfahren wird hier in vollem Umfang Bezug genommen.

Die zuvor beschriebenen Adsorptionsmittel eignen sich nicht nur für den Einsatz in einem Verfahren zur katalytischen Oligomerisierung von monoolefinischen Kohlenwasserstoffgemischen, sondern ganz allgemein zur Behandlung von Olefinen oder Olefingemischen zur Verringerung des Gehaltes an Katalysatorgiften. Gegenstand der Erfindung ist daher auch ein Verfahren zur Herstellung reiner Olefine oder Olefingemische, das dadurch gekennzeichnet ist, dass man sie mit einem der zuvor beschriebenen Adsorptionsmittel in Kontakt bringt.

Ein weiterer Gegenstand der Erfindung ist auch die Verwendung eines Adsorptionsmittels, wie zuvor beschrieben, zur Verringerung des Gehaltes an Verunreinigungen in Olefinen, Olefingemischen und olefinhaltigen Kohlenwasserstoffgemischen.

Die Erfindung wird anhand der folgenden nichteinschränkenden Beispiele näher erläutert.

### Beispiele

Für die folgenden Beispiele wurde ein teilhydriertes Kohlenwasserstoffgemisch verwendet, welches enthält:

| | |
|---|---|
| 31 Gew.-% | trans-2-Buten, |
| 26 Gew.-% | 1-Buten, |
| 26 Gew.-% | n-Butan und Isobutan, |
| 16 Gew.-% | cis-2-Buten, |
| 1 Gew.-% | Isobuten, |
| <1 Gew.-ppm | 1,2-Butadien, |
| <1 Gew.-ppm | 1,3-Butadien, |
| <1 Gew.-ppm | Vinylacetylen, |
| 20 bis 40 Gew.-ppm | Wasser, |
| 1 bis 3 Gew.-ppm | Methanol, |
| 1 bis 14 Gew.-ppm | Ethanol, |
| 1 bis 2 Gew.-ppm | i-Propanol, |
| <1 Gew.-ppm | n-Propanol, |
| 6 bis 35 Gew.-ppm | tert.-Butanol, |
| <1 bis 14 Gew.-ppm | sec.-Butanol, |
| <1 bis 6 Gew.-ppm | i-Butanol, |
| <1 bis 2 Gew.-ppm | n-Butanol, |
| <1 Gew.-ppm | Formaldehyd, |
| <1 bis 3 Gew.-ppm | Acetaldehyd, |
| <1 bis 2 Gew.-ppm | Propionaldehyd, |
| <1 bis 6 Gew.-ppm | Butyraldehyd, |
| 1 bis 13 Gew.-ppm | Aceton. |

### Beispiel 1

Teilhydrierter C₄-Schnitt der oben angegebenen Zusammensetzung wird mit etwa 10 kg/h durch einen 5 l Adsorber geleitet, der ein Festbett aus 5 1 aktivem Al₂O₃ (3 mm Kugeln; Fa. Alcoa, Pittsburgh, USA) mit einer BET-Oberfläche, bestimmt nach DIN 66131, von 400 m²/g enthält. Der Druck im Adsorber beträgt etwa 4 bar, die Temperatur liegt bei etwa 15 °C. Zur Bestimmung der Durchbruchskapazität des Adsorbers und des Gehalts an gebildeten Oligomeren wird der Adsorberaustrag gaschromatographisch untersucht. Die in dem teilhydrierten C₄-Schnitt enthaltenen, als Katalysatorgifte wirksamen Alkohole, Aldehyde und Ketone sind in dem aus dem Adsorber tretenden Gasstrom insgesamt nur noch in einer Menge von höchstens 1 Gew.-ppm enthalten. Das aus dem Adsorber austretende Kohlenwasserstoffgemisch kann so zur katalytischen Oligomerisierung eingesetzt werden.

Als Durchbruchskapazität wird ein Schadstoffschwellenwert des aus dem Adsorber austretenden Gasstroms definiert.

Nach Erreichen der Durchbruchskapazität (mehr als 10 Gew.-ppm Katalysatorgifte im Austritts-Kohlenwasserstoffgemisch) wird der Adsorber zunächst durch Spülen mit N₂ trockengeblasen und anschließend durch Spülen mit Sauerstoff bei einer Temperatur von 40 bis 500 °C und einem Druck von 1 bis 2 bar regeneriert und erneut bis zum Erreichen der Durchbruchskapazität unter den zuvor angegebenen Bedingungen eingesetzt (2. Zyklus). Der Anteil an Oligomerenbildung und die Durchbruchskapazitäten der beiden Zyklen sind in Tabelle 1 wiedergegeben.

### Vergleichsbeispiel 1

Es wird analog zu Beispiel 1 verfahren, wobei als Adsorptionsmittel ein Zeolith vom Typ 13X (3 mm Stränge, Fa. Universal Oil Products) mit einer BET-Oberfläche, bestimmt nach DIN 66131, von 520 m²/g eingesetzt wird. Der Anteil an Oligomerenbildung und die Durchbruchskapazitäten sind ebenfalls in Tabelle 1 angegeben.

**Tabelle 1**

| | Beispiel 1 | Vergleichsbeispiel 1 |
|---|---|---|
| Durchbruchskapazität 1. Zyklus [Gew.-%] | 5,0 | 9,4 |
| Oligomerenbildung 1. Zyklus [Gew.-%]^{a)} | <0,05 | 0,52 |
| Durchbruchskapazität 2. Zyklus [Gew.-%] | 6,0 | 7,9 |
| Oligomerenbildung 2. Zyklus [Gew.-%] | <0,05 | 0,5 |

| | | |
|---|---|---|
| a) bezogen auf Buten-Gehalt; bestimmt 2 Stunden nach Anfahren des Adsorbers | | |

## Patentansprüche

1. Verfahren zur katalytischen Oligomerisierung von in einem Kohlenwasserstoffgemisch enthaltenen kurzkettigen Monoolefinen, wobei man das Gemisch mit einem Adsorptionsmittel in Kontakt bringt und katalytisch oligomerisiert und wobei ein Kohlenwasserstoffgemisch eingesetzt wird, das mindestens ein Monoolefin mit 4 bis 8 Kohlenstoffatomen aufweist und das ausschließlich oder einen hohen Anteil unverzweigte(r) Monoolefine enthält und wobei als Adsorptionsmittel ein Aluminiumoxid oder ein aluminiumoxidhaltiger Feststoff eingesetzt wird.

2. Verfahren nach Anspruch 1, **dadurch gekennzeichnet, dass** als monoolefinhaltiges Kohlenwasserstoffgemisch ein C₄-Schnitt eingesetzt wird.

3. Verfahren nach Anspruch 2, **dadurch gekennzeichnet, dass** als monoolefinhaltiges Kohlenwasserstoffgemisch ein Raffinat II eingesetzt wird.

4. Verfahren nach Anspruch 2, **dadurch gekennzeichnet, dass** als monoolefinhaltiges Kohlenwasserstoffgemisch ein C₄-Schnitt eingesetzt wird, der 10 bis 90 Gew.-% Butene und 10 bis 90 Gew.-% Butane enthält, wobei die Buten-Fraktion die folgenden Kohlenwasserstoffe enthält: 1 bis 99 Gew.-% trans-2-Buten, 1 bis 50 Gew.-% 1-Buten, 1 bis 50 Gew.-% cis-2-Buten und 1 bis 5 Gew.-Isobuten.

5. Verfahren nach Anspruch 2, **dadurch gekennzeichnet, dass** das Adsorptionsmittel zusätzlich noch wenigstens eine Alkalimetall- und/oder Erdalkalimetallverbindung aufweist.

6. Verfahren nach einem der vorhergehenden Ansprüche, **dadurch gekennzeichnet, dass** ein Adsorptionsmittel mit einer spezifischen Oberfläche, bestimmt nach BET, im Bereich von 10 bis 2 000 m²/g eingesetzt wird.

7. Verfahren nach einem der vorhergehenden Ansprüche, **dadurch gekennzeichnet, dass** das Adsorptionsmittel im Wesentlichen keine katalytische Aktivität zur Oligomerisierung von Olefinen aufweist.

8. Verfahren nach einem der vorhergehenden Ansprüche, **dadurch gekennzeichnet, dass** das Adsorptionsmittel in Form eines Festbettes oder eines Wanderbettes eingesetzt wird.

9. Verfahren nach einem der vorhergehenden Ansprüche, **dadurch gekennzeichnet, dass** das monoolefinhaltige Kohlenwasserstoffgemisch in gasförmiger, flüssiger oder überkritischer Phase mit dem Adsorptionsmittel in Kontakt gebracht wird.

10. Verfahren nach einem der vorhergehenden Ansprüche, **dadurch gekennzeichnet, dass** das zur Oligomerisierung eingesetzte monoolefinhaltige Kohlenwasserstoffgemisch einen Gehalt an sauerstoff-, stickstoff- und/oder schwefelhaltigen Verunreinigungen von höchstens 10 ppm, bevorzugt höchstens 1 ppm, aufweist.

11. Verfahren nach einem der vorhergehenden Ansprüche umfassend die Regenerierung der eingesetzten Adsorptionsmittel durch Behandlung mit Sauerstoff, sauerstoffhaltigen Gasgemischen, Sauerstoff liefernden Verbindungen, Stickoxiden oder Gemischen davon.

## Claims

1. A process for the catalytic oligomerization of short-chain monoolefins present in a hydrocarbon mixture by contacting the mixture with an adsorbent and catalytic oligomerization of the mixture, where a hydrocarbon mixture is used which contains at least one monoolefin having 4 to 8 carbon atoms and which contains exclusively unbranched monoolefins or a high proportion of unbranched monoolefins, and where the adsorbent used is one aluminum oxide or one solid containing an aluminum oxide.

2. A process as claimed in claim 1, wherein the monoolefin-containing hydrocarbon mixture used is a C₄ cut.

3. A process as claimed in claim 2, wherein the monoolefin-containing hydrocarbon mixture used is a raffinate II.

4. A process as claimed in claim 2, wherein the monoolefin-containing hydrocarbon mixture used is a C₄ cut which contains from 10 to 90% by weight of butenes and from 10 to 90% by weight of butanes, the butene fraction containing the following hydrocarbons: from 1 to 99% by weight of trans-2-butene, from 1 to 50% by weight of 1-butene, from 1 to 50% by weight of cis-2-butene and from 1 to 5% by weight of isobutene.

5. A process as claimed in claim 2, wherein the adsorbent additionally comprises at least one alkali metal compound and/or alkaline earth metal compound.

6. A process as claimed in any of the preceding claims, wherein the adsorbent used has a specific surface area, determined by the BET method, in the range from 10 to 2000 m²/g.

7. A process as claimed in any of the preceding claims, wherein the adsorbent has essentially no catalytic activity for the oligomerization of olefins.

8. A process as claimed in any of the preceding claims, wherein the adsorbent is used in the form of a fixed bed or a moving bed.

9. A process as claimed in any of the preceding claims, wherein the monoolefin-containing hydrocarbon mixture is contacted with the adsorbent in the gas, liquid or supercritical phase.

10. A process as claimed in any of the preceding claims, wherein the monoolefin-containing hydrocarbon mixture used for the oligomerization contains oxygen-, nitrogen- and/or sulfur-containing impurities in an amount of 10 ppm or less, preferably 1 ppm or less.

11. A process as claimed in any of the preceding claims, comprising the adsorbent used being regenerated by treatment with oxygen, oxygen-containing gas mixtures, oxygen-supplying compounds, nitrogen oxides or mixtures thereof.

## Revendications

1. Procédé d'oligomérisation catalytique de mono-oléfines à chaîne courte contenues dans un mélange d'hydrocarbures, dans lequel on met le mélange en contact avec un adsorbant et on réalise l'oligomérisation par voie catalytique, et dans lequel on met en oeuvre un mélange d'hydrocarbures qui présente au moins une mono-oléfine possédant 4 à 8 atomes de carbone et qui contient exclusivement des mono-oléfines non ramifiées ou une proportion importante de mono-oléfines non ramifiées et dans lequel on met en oeuvre en tant qu'adsorbant un oxyde d'aluminium ou un solide contenant un oxyde d'aluminium.

2. Procédé selon la revendication 1, **caractérisé en ce que** l'on met en oeuvre, en tant que mélange d'hydrocarbures contenant des mono-oléfines, une coupe en C₄ .

3. Procédé selon la revendication 2, **caractérisé en ce que** l'on met en oeuvre, en tant que mélange d'hydrocarbures contenant des mono-oléfines, un raffinat II.

4. Procédé selon la revendication 2, **caractérisé en ce que** l'on met en oeuvre, en tant que mélange d'hydrocarbures contenant des mono-oléfines, une coupe en C₄, qui contient 10% à 90% en poids de butènes et 10% à 90% en poids de butanes, dans laquelle la fraction de butènes contient les hydrocarbures suivants : 1% à 99% en poids de trans-2-butène, 1% à 50% en poids de 1-butène, 1% à 50% en poids de cis-2-butène et 1% à 5% en poids d'isobutène.

5. Procédé selon la revendication 2, **caractérisé en ce que** l'adsorbant présente en outre au moins un composé de métal alcalin et/ou de métal alcalino-terreux.

6. Procédé selon l'une quelconque des revendications précédentes, **caractérisé en ce que** l'on met en oeuvre un adsorbant présentant une surface spécifique, déterminée selon BET, allant de 10 m²/g à 2 000 m²/g.

7. Procédé selon l'une quelconque des revendications précédentes, **caractérisé en ce que** l'adsorbant ne présente pratiquement pas d'activité catalytique pour l'oligomérisation d'oléfines.

8. Procédé selon l'une quelconque des revendications précédentes, **caractérisé en ce que** l'on met en oeuvre l'adsorbant sous la forme d'un lit fixe ou d'un lit mobile.

9. Procédé selon l'une quelconque des revendications précédentes, **caractérisé en ce que** l'on met le mélange d'hydrocarbures contenant des mono-oléfines en phase gazeuse, liquide ou surcritique en contact avec l'adsorbant.

10. Procédé selon l'une quelconque des revendications précédentes, **caractérisé en ce que** le mélange d'hydrocarbures contenant des mono-oléfines mis en oeuvre pour l'oligomérisation présente une teneur en impuretés contenant de l'oxygène, de l'azote et/ou du soufre d'au maximum 10 ppm, de préférence d'au maximum 1 ppm.

11. Procédé selon l'une quelconque des revendications précédentes, comprenant la régénération de l'adsorbant mis en oeuvre au moyen d'un traitement avec de l'oxygène, des mélanges gazeux contenant de l'oxygène, des composés fournissant de l'oxygène, des oxydes d'azote ou des mélanges de ceux-ci.
